# EUROPEAN PATENT SPECIFICATION

(11) **EP 4 081 277 B1**
(45) Date of publication and mention of the grant of the patent: **30.10.2024**
(21) Application number: 19957853.5
(22) Date of filing: 25.12.2019
(51) Int. Cl.: A61M 1/14, A61M 1/28, A61M 1/16, F15B 1/02, F15B 1/04

(54) **PRESSURE VESSEL, LIQUID MIXING EQUIPMENT, LIQUID MIXING SYSTEM, METHOD FOR PREPARING SOLUTION, CONTROL SYSTEM AND COMPUTER READABLE PROGRAM CARRIER**
DRUCKBEHÄLTER, FLÜSSIGKEITSMISCHVORRICHTUNG, FLÜSSIGKEITSMISCHSYSTEM, VERFAHREN ZUR HERSTELLUNG EINER LÖSUNG, STEUERUNGSSYSTEM UND COMPUTERLESBARER PROGRAMMTRÄGER
RÉCIPIENT SOUS PRESSION, ÉQUIPEMENT DE MÉLANGE DE LIQUIDE, SYSTÈME DE MÉLANGE DE LIQUIDE, PROCÉDÉ DE PRÉPARATION DE SOLUTION, SYSTÈME DE COMMANDE ET SUPPORT DE PROGRAMME LISIBLE PAR ORDINATEUR

(43) Date of publication of application: 02.11.2022
(73) Proprietor: Fresenius Medical Care Deutschland GmbH, 61352 Bad Homburg (DE)
(72) Inventor: JOR, Kee, Hong Kong (CN); LIU, Xiang, Shanghai 200438 (CN)
(74) Representative: Bobbert & Partner Patentanwälte PartmbB
(86) International application number: PCT/CN2019/128343
(87) International publication number: WO 2021/128067

(56) References cited:
- WO-A1-2019/119414
- CN-A- 105 034 604
- CN-A- 106 573 095
- CN-A- 107 645 960
- CN-A- 108 121 093
- CN-A- 109 550 095
- US-A1- 2017 290 970

## Description

### Technical Field

The disclosure relates to a pressure vessel for liquid mixing, a liquid mixing equipment comprising the pressure vessel, a liquid mixing system comprising the pressure vessel or the liquid mixing equipment, a method for preparing a solution using the pressure vessel, the liquid mixing equipment or the liquid mixing system, a corresponding control system and a corresponding computer readable program carrier.

### Background Art

It is known that, in many fields, for example in the medical field, it is necessary to prepare solutions according to well-specified dosages in order to carry out specific treatments. Such is for example the case of dialysis solution for dialysis treatment. The dialysis treatment is a process for purifying blood of a person whose kidney is not working normally.

Nowadays, the dialysis solution is often prepared just before dialysis sessions. To do so, solid substance for example in a form of powders, granules or tablets or mixtures thereof is placed in a container and can be dissolved by liquid, for example reverse osmosis (RO) water to obtain a solution which may be a dialysis solution or an intermediate solution for preparing the dialysis solution. For example, liquid concentrates like bicarbonate and sodium saturated solution can be prepared online from the corresponding solid substances. During the dissolving process, refilling of the liquid into the container is controlled to obtain a desired solution flow toward a downstream dialysate mixing path.

The container may be made of rigid or soft plastic material. For the soft container, the refilling process can be done by a pressure-controlled method. Specifically, the soft container is first filled with the liquid and followed by refilling cycles based on predetermined upper and lower fluid pressure set points.

However, the pressure-controlled method cannot be used for the rigid container in a closed hydraulic system design. When the liquid in the closed container is dispensed to the dialysate mixing path, excessive pressure change will act on a downstream liquid concentrate pump, which may damage the concentrate pump and lead to a mixing error.

Relevant prior art is for instance disclosed in document US2017/290970 A1.

### Summary of the Disclosure

In view of at least one of the problems existing in the prior art, an object of the present disclosure is to provide a pressure vessel for liquid mixing, a liquid mixing equipment comprising the pressure vessel, a liquid mixing system comprising the pressure vessel or the liquid mixing equipment, a method for preparing a solution using the pressure vessel, the liquid mixing equipment or the liquid mixing system, a corresponding control system and a corresponding computer readable program carrier.

For achieving this object, according to a first aspect, provided is a pressure vessel for liquid mixing, comprising: a housing; a deformable member configured to define a chamber in combination with at least one portion of the housing; an inlet port fluidly communicated with the chamber; an outlet port fluidly communicated with the chamber; an elastic biasing means configured to interact with the deformable member to make a volume of the chamber variable; and an air detection means configured to detect the presence of air within the chamber.

According to an optional embodiment of the present disclosure, the deformable member is formed by a flexible diaphragm; and/or the inlet port and the outlet port are disposed opposite to each other.

According to an optional embodiment of the present disclosure, the elastic biasing means comprises a spring, preferably a conical coil spring, or is configured in a manner of compressed gas, preferably compressed air; and/or the air detection means comprises a pair of air detection electrodes extending into the chamber.

According to an optional embodiment of the present disclosure, an inner space of the chamber is configured to generate a fluid flow for facilitating hygiene control in a cleaning and/or disinfection process; and/or the elastic biasing means is adjustable to regulate the volume of the chamber in operation.

According to an optional embodiment of the present disclosure, the inner space of the chamber is compressed by a force of the elastic biasing means to some extent at an initial stage; preferably, the inner space of the chamber is configured to have initially a U-shaped section along a direction of the elastic biasing means acting on the deformable member and extending the inlet port and the outlet port, and a bottom portion of the U-shaped section is defined by the deformable member.

According to an optional embodiment of the present disclosure, the air detection means is disposed adjacent to an outer end of a first leg of the U-shaped section; and/or the inlet port is disposed adjacent to a corner between the first leg and the bottom portion of the U-shaped section; and/or the outlet port is disposed adjacent to a middle point of a second leg of the U-shaped section opposite to the first leg.

According to a second aspect, provided is a liquid mixing equipment comprising the pressure vessel described above.

According to a third aspect, provided is a liquid mixing system comprising the pressure vessel described above or the liquid mixing equipment described above.

According to an optional embodiment of the present disclosure, the liquid mixing system further comprises: a canister, in particular a rigid canister, for receiving a substance to be dissolved or diluted by a first liquid to prepare a solution, preferably a saturated solution, wherein the outlet port of the pressure vessel is fluidly connected to the canister; and a mixing chamber fluidly connected to the canister to receive the solution; preferably, the inlet port of the pressure vessel is positioned higher than the outlet port of the pressure vessel in an operation state.

According to an optional embodiment of the present disclosure, the liquid mixing system further comprises: a first flow path fluidly connected to the inlet port of the pressure vessel to allow for filling the canister with the first liquid via the first flow path and the pressure vessel; and/or a second flow path fluidly connected to the canister to allow for filling the canister with a second liquid; and/or a third flow path fluidly connected to the canister to allow for transferring the solution to the mixing chamber; and/or a fourth flow path fluidly connected to the mixing chamber to supply a third liquid to the mixing chamber.

According to an optional embodiment of the present disclosure, the liquid mixing system further comprises: a pressure detection means configured to detect a pressure within the first flow path; a first valve disposed in the first flow path upstream of the pressure detection means; and an air exhausting means fluidly connected to the first flow path between the first valve and the pressure vessel.

According to an optional embodiment of the present disclosure, the air exhausting means comprises a second valve and an air outlet; and/or the canister has a top and a bottom in the operation state, and the pressure vessel is fluidly connected to a top inlet port of the canister to allow for filling the canister with the first liquid via the top of the canister.

According to an optional embodiment of the present disclosure, the liquid mixing system further comprises a third valve disposed in the second flow path; and/or the second flow path is configured to allow for filling the canister with the second liquid via the bottom of the canister.

According to an optional embodiment of the present disclosure, the liquid mixing system further comprises: a solution pump disposed in the third flow path to pump the solution to the mixing chamber; and/or a fourth valve, preferably a check valve, disposed in the fourth flow path.

According to an optional embodiment of the present disclosure, at least two of the first flow path, the second flow path and the fourth path are fluidly connected to a common liquid source; and/or at least one of the first liquid, the second liquid and the third liquid is reverse osmosis water; and/or the substance is in form of powder, granules, tablets or mixtures thereof; and/or the substance comprises sodium chloride and/or bicarbonate, in particular sodium bicarbonate.

According to a fourth aspect, provided is a method for preparing a solution by using the pressure vessel described above, the liquid mixing equipment described above or the liquid mixing system described above, comprising: detecting presence of air within the chamber of the pressure vessel; exhausting the air through the pressure vessel or out of the liquid mixing system in case of the presence of the air; and executing liquid supply through the pressure vessel to obtain the solution.

According to an optional embodiment of the present disclosure, the method further comprises: keeping a liquid pressure of the pressure vessel within a predetermined range.

According to an optional embodiment of the present disclosure, the method further comprises: stopping liquid supply toward the pressure vessel when the liquid pressure is within the predetermined range; and/or executing liquid supply toward the pressure vessel until the liquid pressure of the pressure vessel goes beyond the predetermined range.

According to a fifth aspect, provided is a control system configured to control the liquid mixing system described above to execute the method described above.

According to a sixth aspect, provided is a computer readable program carrier storing program instructions, wherein the method described above is implemented when the program instructions are executed by a processor.

According to the present disclosure, the solution pump can draw the solution in a substantially constant flow toward the mixing chamber so as to obtain the solution having a desired concentration. In addition, the solution pump can operate at a relatively stable pressure, which is very advantageous.

### Brief Description of the Drawings

The disclosure and advantages thereof will be further understood by reading the following detailed description of some preferred exemplary embodiments with reference to the drawings in which:
Fig.1 shows a schematic sectional view of a pressure vessel according to an exemplary embodiment of the present disclosure in a non-operation state.
Fig.2 shows a schematic sectional view of the pressure vessel as shown in Fig.1 in an operation state.
Fig.3 shows a sectional view of the pressure vessel according to an exemplary embodiment of the present disclosure.
Fig.4 shows a left side view of the pressure vessel as shown in Fig.3.
Fig.5 shows a schematic view of a liquid mixing system using the pressure vessel according to an exemplary embodiment of the present disclosure.
Fig.6 shows a first operation phase of the liquid mixing system as shown in Fig.5.
Fig.7 shows a second operation phase of the liquid mixing system as shown in Fig.5.

### Detailed Description of Preferred Embodiments

Some exemplary embodiments of the present disclosure will be described hereinafter in more details with reference to the drawings to better understand the basic concept of the present disclosure.

According to an aspect of the present disclosure, herein firstly proposed is a pressure vessel for liquid mixing.

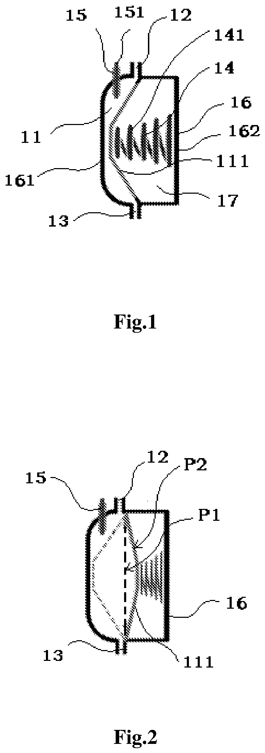

Fig. 1 shows a schematic sectional view of the pressure vessel 1 according to an exemplary embodiment of the present disclosure in a non-operation state.

As shown in Fig.1, the pressure vessel 1 may comprise: a chamber 11, an inlet port 12 fluidly communicated with the chamber 11, an outlet port 13 fluidly communicated with the chamber 11, an elastic biasing means 14 configured to interact with a deformable member 111 of the chamber 11 such that a volume of the chamber 11 is variable, and an air detection means 15 configured to detect the presence of air within the chamber 11. By means of the air detection means 15, it can be detected if the chamber 11 is filled fully with the liquid.

According to an exemplary embodiment of the present disclosure, the deformable member 111 of the chamber 11 may be formed by a flexible diaphragm. Preferably, the inlet port 12 and the outlet port 13 may be disposed opposite to each other, as shown in Fig.1.

As can be seen from Fig.1, the pressure vessel 1 may comprise a housing 16, within which the deformable member 111 of the chamber 11 and the elastic biasing means 14 may be disposed, and the chamber 11 may be defined partially by a first portion 161 of the housing 16. In particular, the chamber 11 may be defined commonly by the first portion 161 of the housing 16 and the deformable member 111 of the chamber 11.

According to an exemplary embodiment of the present disclosure, the elastic biasing means 14 may be configured to comprise a spring 141, preferably a conical coil spring, as shown in Fig.1.

As another exemplary embodiment of the present disclosure, the elastic biasing means 14 also may be configured in a manner of compressed gas, for example compressed air, filled into a chamber 17 defined by a second portion 162 of the housing 16 and the deformable member 111 of the chamber 11.

According to an exemplary embodiment of the present disclosure, the air detection means 15 may be configured to comprise a pair of air detection electrodes 151 (only one of which is shown in Fig.1) extending into the chamber 11. It is possible to detect presence of air within the chamber 11 by detecting conductivity within the chamber 11 using the pair of air detection electrodes 151. Of course, it may be understood by the skilled person in the art that the air detection means 15 also may be configured based on different technical principles and thus the air detection means 15 is not limited thereto.

When liquid, for example RO water is filled into the chamber 11 so as to increase pressure within the chamber 11 through the inlet port 12, the deformable member 111 will be displaced toward the elastic biasing means 14 so as to make the volume of the chamber 11 become bigger, and vice versa.

Fig.2 shows a schematic sectional view of the pressure vessel 1 shown in Fig.1 in an operation state.

As shown Fig.2, the deformable member 111 is displaced to a first position (schematically shown in a dashed line) when the pressure within the chamber 11 reaches a first pressure P1, for example 100 mbar, and the deformable member 111 is further displaced to a second position when the pressure within the chamber 11 further increases to a second pressure P2, for example 130 mbar, higher than the first pressure P1.

In particular for medical applications, it may be desired that the chamber 11 can be cleaned easily, for example before use. To this end, according to an exemplary embodiment of the present disclosure, an inner space of the chamber 11 may be configured to be able to generate a fluid flow for facilitating hygiene control in a cleaning and/or disinfection process. For example, the inner space of the chamber 11 may be configured to have no dead zone which cannot be cleaned easily.

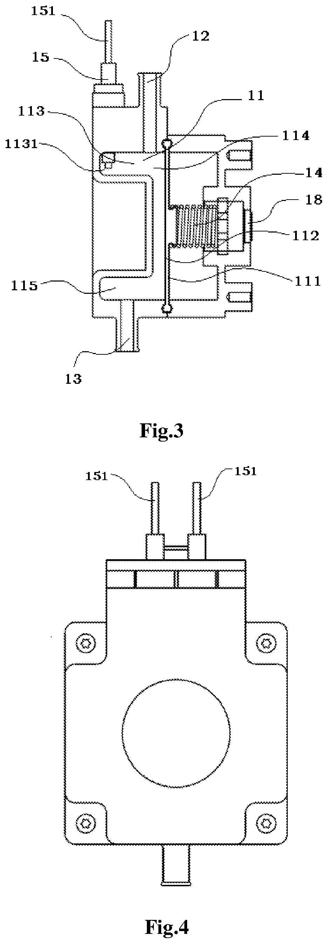

Fig.3 shows a sectional view of the pressure vessel 1 according to an exemplary embodiment of the present disclosure.

Preferably, the inner space of the chamber 11 may be compressed by a force of the elastic biasing means 14 to some extent at an initial stage.

As shown in Fig.3, the inner space of the chamber 11 may be configured to have initially a U-shaped section which is taken along a direction of the elastic biasing means 14 acting on the deformable member 111 of the chamber 11 and extends through the inlet port 12 and the outlet port 13, and a bottom portion 112 of the U-shaped section may be defined by the deformable member 111 of the chamber 11.

According to an exemplary embodiment of the present disclosure, as also can be seen from Fig.3, the air detection means 15 may be disposed adjacent to an outer end 1131 of a first leg 113 of the U-shaped section. Also, the inlet port 12 may be disposed adjacent to a corner 114 between the first leg 113 and the bottom portion 112 of the U-shaped section. Further, the outlet port 13 may be disposed adjacent to a middle point of a second leg 115 of the U-shaped section opposite to the first leg 113.

According to an exemplary embodiment of the present disclosure, the elastic biasing means 14 may be configured to be able to be adjusted to adjust the volume of the chamber 11 in operation. Specifically, elastic deformation characteristics of the elastic biasing means 14 can be adjusted so as to change deformation characteristics of the deformable member 111 at the same pressure within the chamber 11, thereby changing the volume of the chamber 11 in operation.

As further shown in Fig.3, an adjusting screw 18 may be provided to adjust the elastic biasing means 14.

Fig.4 shows a left side view of the pressure vessel 1 as shown in Fig.3. The pair of air detection electrodes 151 can be seen in Fig.4.

According to another aspect of the present disclosure, further proposed is a liquid mixing system comprising the pressure vessel 1.

Below, the liquid mixing system will be described.

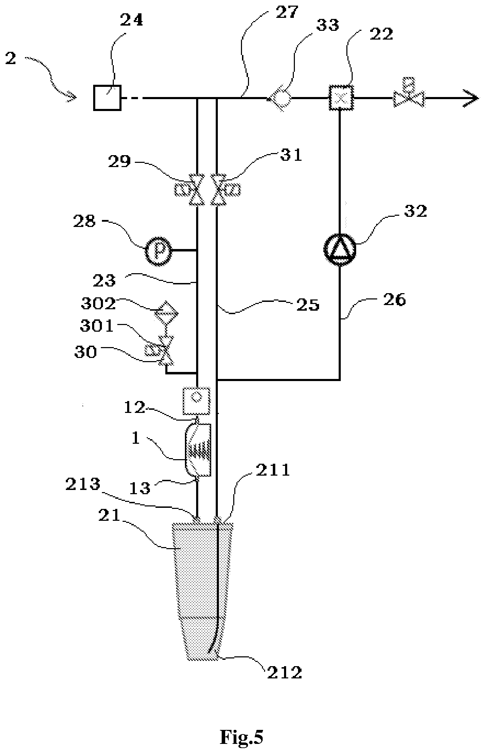

Fig.5 shows a schematic view of the liquid mixing system 2 using the pressure vessel 1 according to an exemplary embodiment of the present disclosure.

As shown in Fig.5, the liquid mixing system 2 may further comprise: a rigid canister 21 for receiving a substance, for example bicarbonate powder, to be dissolved by a first liquid so as to obtain a saturated liquid concentrate, wherein the outlet port 13 of the pressure vessel 1 is fluidly connected to the canister 21 and the inlet port 12 of the pressure vessel 1 is positioned higher than the outlet port 13 of the pressure vessel 1 in an operation state; and a mixing chamber 22 fluidly connected to the canister 21 to receive the liquid concentrate.

The substance may be in a form including powder, granules, tablets or mixtures thereof. Further, the substance may comprise bicarbonate, in particular sodium bicarbonate, and/or sodium chloride.

According to an exemplary embodiment of the present disclosure, the liquid mixing system 2 may further comprise a first flow path 23 fluidly connected to the inlet port 12 of the pressure vessel 1 to allow for filling the canister 21 with the first liquid via the first flow path 23 and the pressure vessel 1. In the embodiment shown in Fig.5, the first liquid is transferred into the canister 21 from a source 24.

According to an exemplary embodiment of the present disclosure, the liquid mixing system 2 may further comprise a second flow path 25 fluidly connected to the canister 21 to allow for filling the canister 21 with a second liquid.

According to an exemplary embodiment of the present disclosure, the liquid mixing system 2 may further comprise a third flow path 26 fluidly connected to the canister 21 to allow for transferring the liquid concentrate to the mixing chamber 22.

According to an exemplary embodiment of the present disclosure, the liquid mixing system 2 may further comprise a fourth flow path 27 fluidly connected to the mixing chamber 22 to supply a third liquid to the mixing chamber 22.

Preferably, at least one of the first liquid, the second liquid and the third liquid is water, for example RO water. When the first liquid, the second liquid and the third liquid are the same liquid, they can be supplied from the same source, for example the source 24, as shown in Fig.5.

According to an exemplary embodiment of the present disclosure, the liquid mixing system 2 may further comprise: a pressure detection means 28 configured to detect a pressure within the first flow path 23; a first valve 29 disposed in the first flow path 23 upstream of the pressure detection means 28; and an air exhausting means 30 fluidly connected to the first flow path 23 between the first valve 29 and the pressure vessel 1.

Preferably, the air exhausting means 30 may comprise a second valve 301 and an air outlet 302. Air can be exhausted via the air outlet 302 by opening the second valve 301.

According to an exemplary embodiment of the present disclosure, the canister 21 may have a top 211 and a bottom 212 in the operation state, and the pressure vessel 1 may be fluidly connected to a top inlet port 213 of the canister 21 to allow for filling the canister 21 with the first liquid via the top 211 of the canister 21.

Preferably, the liquid mixing system 2 may further comprise a third valve 31 disposed in the second flow path 25.

Preferably, the second flow path 25 may be configured to allow for filling the canister 21 with the second liquid via the bottom 212 of the canister 21.

According to an exemplary embodiment of the present disclosure, the liquid mixing system 2 may further comprise a concentrate pump 32 disposed in the third flow path 26 to pump the liquid concentrate to the mixing chamber 22.

Preferably, the liquid mixing system 2 may further comprise a fourth valve 33, preferably a check valve, disposed in the fourth flow path 27, in order to only allow for flowing of fluid toward the mixing chamber 22.

The liquid mixing system 2 has been described above illustratively in connection with Fig.5 and then it will be described how to operate the liquid mixing system 2.

According to a further aspect of the present disclosure, proposed is a method for preparing a desired solution using the liquid mixing system 2, at least comprising: detecting presence of air within the chamber 11 of the pressure vessel 1; exhausting the air out of the liquid mixing system 2 in the presence of the air; and executing liquid supply through the pressure vessel 1 to obtain the desired solution.

According to an exemplary embodiment of the present disclosure, the method may further comprise: keeping a liquid pressure within the pressure vessel 1 within a predetermined range.

According to an exemplary embodiment of the present disclosure, the method may further comprise: stopping liquid supply toward the pressure vessel 1 when the liquid pressure within the pressure vessel 1 is within the predetermined range.

According to an exemplary embodiment of the present disclosure, the method may further comprise: executing liquid supply toward the pressure vessel 1 until the liquid pressure within the pressure vessel 1 reaches a upper limit, for example 130 mbar, of the predetermined range, when the liquid pressure within the pressure vessel 1 is below a lower limit, for example 100 mbar, of the predetermined range.

In order to better understand the operation of the liquid mixing system 2, it will be described in connection with the embodiment as shown in Fig.5.

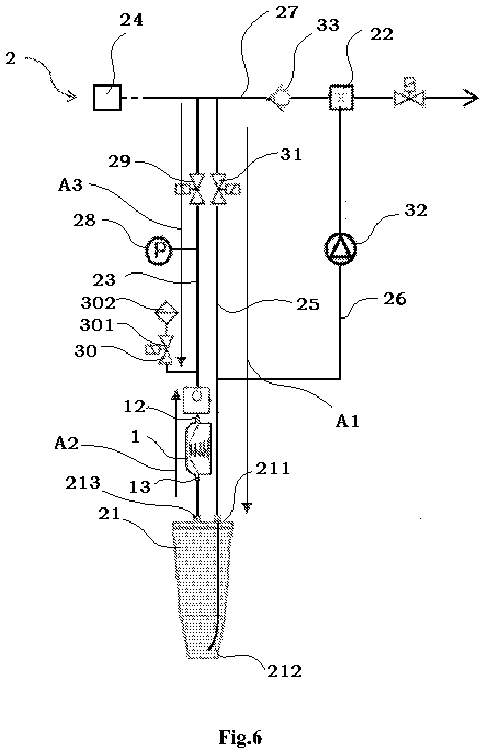

Fig.6 shows a first operation phase of the liquid mixing system 2. As shown in Fig.6, in the first operation phase, the second valve 301 and the third valve 31 are in an open state, the second liquid, for example RO water, is transferred firstly to the bottom 212 of the canister 21 through the second flow path 25 as shown in a first arrow A1 and rises in the canister 21 while dissolving the substance, for example bicarbonate, within the canister 21. As the second liquid rises gradually within the canister 21, air contained in the canister 21 is expelled toward the top 211 of the canister 21 and then into the chamber 11 of the pressure vessel 1 as shown in a second arrow A2. Finally, the air is exhausted via the second valve 301 and the air outlet 302. When the air detection means 15 of the pressure vessel 1 detects the second liquid without any air, the second valve 301 and the third valve 31 are closed and then the first valve 29 is opened to transfer the first liquid, also for example RO water, into the chamber 11 of the pressure vessel 1 as shown in a third arrow A3, until the pressure detected by the pressure detection means 28 reaches the upper limit, for example 130 mbar, of the predetermined range.

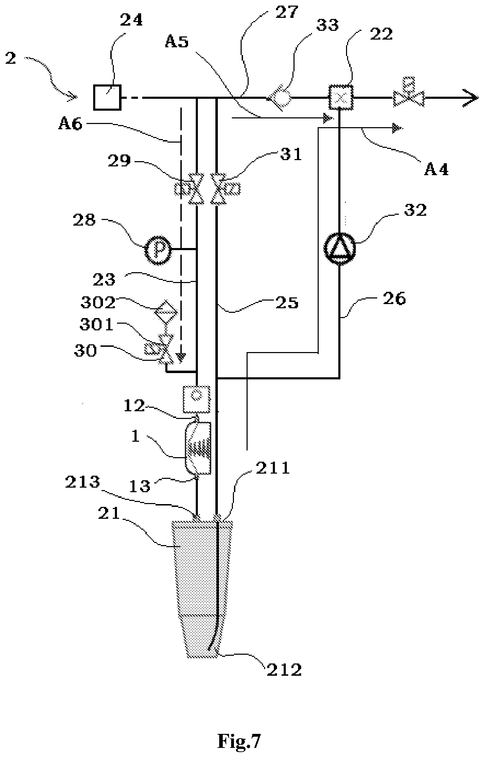

In a second operation phase as shown in Fig.7, the concentrate pump 32 draws the saturated liquid concentrate from the bottom 212 of the canister 21 as shown in a fourth arrow A4 to the mixing chamber 22 to mix with the third liquid, also for example OS water, transferred into the mixing chamber 22 through the fourth flow path 27 as shown in a fifth arrow A5. At the same time, the same amount of the first liquid is dispensed from the pressure vessel 1 to fill the canister 21 from the top inlet port 213 of the canister 21 as shown in a sixth dashed line arrow A6. During the second operation phase, the pressure within the first flow path 23 is monitored by the pressure detection means 28. When the pressure within the first flow path 23 drops to be below the lower limit, for example 100 mbar, of the predetermined range, the first valve 29 is opened to refill the chamber 11 of the pressure vessel 1 with the first liquid until the pressure within the first flow path 23 reaches the upper limit, for example 130 mbar, of the predetermined range. The above processes repeat to deliver the desired solution downstream from the mixing chamber 22.

It may be understood from the above by the skilled person in the art that keeping the pressure within the first flow path 23 within the predetermined range can allow the concentrate pump 32 to draw the saturated liquid concentrate in a substantially constant flow toward the mixing chamber 22 so as to obtain the desired solution having a desired concentration. In addition, the concentrate pump 32 can operate at a relatively stable pressure, which is very advantageous.

According to a further aspect of the present disclosure, proposed is a control system configured to control the liquid mixing system 2 to execute the method described above.

According to another aspect of the present disclosure, proposed is a computer readable program carrier storing program instructions therein, wherein the method described above is achieved when the program instructions are executed by a processor.

While certain embodiments have been described, these embodiments have been presented by way of example only, and are not intended to limit the scope of the disclosure. The invention is defined by the attached claims.

## Claims

1. A pressure vessel (1) for liquid mixing, comprising:
a housing (16);
a deformable member (111) configured to define a chamber (11) in combination with at least one portion of the housing (16);
an inlet port (12) fluidly communicated with the chamber (11);
an outlet port (13) fluidly communicated with the chamber (11);
an elastic biasing means (14) configured to interact with the deformable member (111) to make a volume of the chamber (11) variable; and
an air detection means (15) configured to detect the presence of air within the chamber (11).

2. The pressure vessel (1) according to claim 1, wherein
the deformable member (111) is formed by a flexible diaphragm; and/or
the inlet port (12) and the outlet port (13) are disposed opposite to each other.

3. The pressure vessel (1) according to claim 1 or 2, wherein
the elastic biasing means (14) comprises a spring (141), preferably a conical coil spring, or is configured in a manner of compressed gas, preferably compressed air; and/or
the air detection means (15) comprises a pair of air detection electrodes (151) extending into the chamber (11).

4. The pressure vessel (1) according to any one of claims 1-3, wherein
an inner space of the chamber (11) is configured to generate a fluid flow for facilitating hygiene control in a cleaning and/or disinfection process; and/or
the elastic biasing means (14) is adjustable to regulate the volume of the chamber (11) in operation.

5. The pressure vessel (1) according to claim 4, wherein
the inner space of the chamber (11) is compressed by a force of the elastic biasing means (14) to some extent at an initial stage;
preferably, the inner space of the chamber (11) is configured to have initially a U-shaped section along a direction of the elastic biasing means (14) acting on the deformable member (111) and extending the inlet port (12) and the outlet port (13), and a bottom portion (112) of the U-shaped section is defined by the deformable member (111).

6. The pressure vessel (1) according to claim 5, wherein
the air detection means (15) is disposed adjacent to an outer end (1131) of a first leg (113) of the U-shaped section; and/or
the inlet port (12) is disposed adjacent to a corner (114) between the first leg (113) and the bottom portion (112) of the U-shaped section; and/or
the outlet port (13) is disposed adjacent to a middle point of a second leg (115) of the U-shaped section opposite to the first leg (113).

7. A liquid mixing system (2) comprising the pressure vessel (1) according to any one of claims 1-6.

8. The liquid mixing system (2) according to claim 7, wherein the liquid mixing system (2) further comprises:
a canister (21), in particular a rigid canister, for receiving a substance to be dissolved or diluted by a first liquid to prepare a solution, preferably a saturated solution, wherein the outlet port (13) of the pressure vessel (1) is fluidly connected to the canister (21); and
a mixing chamber (22) fluidly connected to the canister (21) to receive the solution;
preferably, the inlet port (12) of the pressure vessel (1) is positioned higher than the outlet port (13) of the pressure vessel (1) in an operation state.

9. The liquid mixing system (2) according to claim 8, wherein the liquid mixing system (2) further comprises:
a first flow path (23) fluidly connected to the inlet port (12) of the pressure vessel (1) to allow for filling the canister (21) with the first liquid via the first flow path (23) and the pressure vessel (1); and/or
a second flow path (25) fluidly connected to the canister (21) to allow for filling the canister (21) with a second liquid; and/or
a third flow path (26) fluidly connected to the canister (21) to allow for transferring the solution to the mixing chamber (22); and/or
a fourth flow path (27) fluidly connected to the mixing chamber (22) to supply a third liquid to the mixing chamber (22).

10. The liquid mixing system (2) according to claim 9, wherein the liquid mixing system (2) further comprises:
a pressure detection means (28) configured to detect a pressure within the first flow path (23);
a first valve (29) disposed in the first flow path (23) upstream of the pressure detection means (28); and
an air exhausting means (30) fluidly connected to the first flow path (23) between the first valve (29) and the pressure vessel (1).

11. The liquid mixing system (2) according to claim 10, wherein
the air exhausting means (30) comprises a second valve (301) and an air outlet (302); and/or
the canister (21) has a top (211) and a bottom (212) in the operation state, and the pressure vessel (1) is fluidly connected to a top inlet port (213) of the canister (21) to allow for filling the canister (21) with the first liquid via the top (211) of the canister (21).

12. The liquid mixing system (2) according to any one of claims 9-11, wherein
the liquid mixing system (2) further comprises a third valve (31) disposed in the second flow path (25); and/or
the second flow path (25) is configured to allow for filling the canister (21) with the second liquid via the bottom (212) of the canister (21).

13. The liquid mixing system (2) according to any one of claims 9-12, wherein the liquid mixing system (2) further comprises:
a solution pump (32) disposed in the third flow path (26) to pump the solution to the mixing chamber (22); and/or
a fourth valve (33), preferably a check valve, disposed in the fourth flow path (27).

14. The liquid mixing system (2) according to any one of claims 9-13, wherein
at least two of the first flow path (23), the second flow path (25) and the fourth path (27) are fluidly connected to a common liquid source (24); and/or
at least one of the first liquid, the second liquid and the third liquid is reverse osmosis water; and/or
the substance is in form of powder, granules, tablets or mixtures thereof; and/or
the substance comprises sodium chloride and/or bicarbonate, in particular sodium bicarbonate.

15. A method for preparing a solution by using the pressure vessel (1) according to any one of claims 1-6 or the liquid mixing system (2) according to any one of claims 7-14, comprising:
detecting presence of air within the chamber (11) of the pressure vessel (1);
exhausting the air through the pressure vessel (1) or out of the liquid mixing system (2) in case of the presence of the air; and
executing liquid supply through the pressure vessel (1) to obtain the solution.

## Patentansprüche

1. Ein Druckbehälter (1) zum Mischen von Flüssigkeiten, umfassend:
ein Gehäuse (16);
ein verformbares Element (111), das so konfiguriert ist, dass es zusammen mit mindestens einem Teil des Gehäuses (16) eine Kammer (11) definiert;
einen Einlass (12), der in Fluidkommunikation mit der Kammer (11) steht;
einen Auslass (13), der in Fluidkommunikation mit der Kammer (11) steht;
ein elastisches Vorspannelement (14), das so konfiguriert ist, dass es mit dem verformbaren Element (111) interagiert, um ein Volumen der Kammer (11) variabel zu machen; und
ein Luftdetektionsmittel (15), das so konfiguriert ist, dass es das Vorhandensein von Luft innerhalb der Kammer (11) erkennt.

2. Der Druckbehälter (1) nach Anspruch 1, wobei
das verformbare Element (111) durch eine flexible Membran gebildet ist; und/oder
der Einlass (12) und der Auslass (13) einander gegenüberliegend angeordnet sind.

3. Der Druckbehälter (1) nach Anspruch 1 oder 2, wobei
das elastische Vorspannelement (14) eine Feder (141), vorzugsweise eine konische Schraubenfeder, umfasst oder in Form eines komprimierten Gases, vorzugsweise komprimierter Luft, konfiguriert ist; und/oder
das Luftdetektionsmittel (15) ein Paar Luftdetektionselektroden (151) umfasst, die sich in die Kammer (11) erstrecken.

4. Der Druckbehälter (1) nach einem der Ansprüche 1 bis 3, wobei
ein Innenraum der Kammer (11) so konfiguriert ist, dass er einen Fluidstrom erzeugt, um die Hygienekontrolle in einem Reinigungs- und/oder Desinfektionsprozess zu erleichtern; und/oder
das elastische Vorspannelement (14) einstellbar ist, um das Volumen der Kammer (11) im Betrieb zu regeln.

5. Der Druckbehälter (1) nach Anspruch 4, wobei
der Innenraum der Kammer (11) in einem anfänglichen Stadium durch eine Kraft des elastischen Vorspannelements (14) bis zu einem gewissen Grad komprimiert wird;
vorzugsweise der Innenraum der Kammer (11) so konfiguriert ist, dass er anfangs einen U-förmigen Querschnitt entlang einer Richtung des auf das verformbare Element (111) wirkenden elastischen Vorspannelements (14) hat und den Einlass (12) und den Auslass (13) verlängert, wobei ein Bodenabschnitt (112) des U-förmigen Querschnitts durch das verformbare Element (111) definiert ist.

6. Der Druckbehälter (1) nach Anspruch 5, wobei
das Luftdetektionsmittel (15) benachbart zu einem äußeren Ende (1131) eines ersten Schenkels (113) des U-förmigen Querschnitts angeordnet ist; und/oder
der Einlass (12) benachbart zu einer Ecke (114) zwischen dem ersten Schenkel (113) und dem Bodenabschnitt (112) des U-förmigen Querschnitts angeordnet ist; und/oder
der Auslass (13) benachbart zu einem Mittelpunkt eines zweiten Schenkels (115) des U-förmigen Querschnitts gegenüber des ersten Schenkels (113) angeordnet ist.

7. Ein Flüssigkeitsmischsystem (2), das den Druckbehälter (1) nach einem der Ansprüche 1 bis 6 umfasst.

8. Das Flüssigkeitsmischsystem (2) nach Anspruch 7, wobei das Flüssigkeitsmischsystem (2) ferner umfasst:
einen Kanister (21), insbesondere einen starren Kanister, zur Aufnahme einer Substanz, die von einer ersten Flüssigkeit gelöst oder verdünnt werden soll, um eine Lösung zuzubereiten, vorzugsweise eine gesättigte Lösung, wobei der Auslass (13) des Druckbehälters (1) in Fluidkommunikation mit dem Kanister (21) steht; und
eine Mischkammer (22), die in Fluidkommunikation mit dem Kanister (21) steht, um die Lösung aufzunehmen;
vorzugsweise ist der Einlass (12) des Druckbehälters (1) in einem Betriebszustand höher positioniert als der Auslass (13) des Druckbehälters (1).

9. Das Flüssigkeitsmischsystem (2) nach Anspruch 8, wobei das Flüssigkeitsmischsystem (2) ferner umfasst:
einen ersten Flussweg (23), der in Fluidkommunikation mit dem Einlass (12) des Druckbehälters (1) steht, um das Befüllen des Kanisters (21) mit der ersten Flüssigkeit über den ersten Flussweg (23) und den Druckbehälter (1) zu ermöglichen; und/oder
einen zweiten Flussweg (25), der in Fluidkommunikation mit dem Kanister (21) steht, um das Befüllen des Kanisters (21) mit einer zweiten Flüssigkeit zu ermöglichen; und/oder
einen dritten Flussweg (26), der in Fluidkommunikation mit dem Kanister (21) steht, um die Übertragung der Lösung in die Mischkammer (22) zu ermöglichen; und/oder
einen vierten Flussweg (27), der in Fluidkommunikation mit der Mischkammer (22) steht, um eine dritte Flüssigkeit der Mischkammer (22) zuzuführen.

10. Das Flüssigkeitsmischsystem (2) nach Anspruch 9, wobei das Flüssigkeitsmischsystem (2) ferner umfasst:
ein Druckerfassungsmittel (28), das so konfiguriert ist, dass es einen Druck innerhalb des ersten Flusswegs (23) erfasst;
ein erstes Ventil (29), das im ersten Flussweg (23) stromaufwärts des Druckerfassungsmittels (28) angeordnet ist; und
ein Luftauslassmittel (30), das in Fluidkommunikation mit dem ersten Flussweg (23) zwischen dem ersten Ventil (29) und dem Druckbehälter (1) steht.

11. Das Flüssigkeitsmischsystem (2) nach Anspruch 10, wobei
das Luftauslassmittel (30) ein zweites Ventil (301) und einen Luftauslass (302) umfasst; und/oder
der Kanister (21) in dem Betriebszustand eine Oberseite (211) und eine Unterseite (212) aufweist, und der Druckbehälter (1) in Fluidkommunikation mit einer oberen Einlassöffnung (213) des Kanisters (21) steht, um das Befüllen des Kanisters (21) mit der ersten Flüssigkeit über die Oberseite (211) des Kanisters (21) zu ermöglichen.

12. Das Flüssigkeitsmischsystem (2) nach einem der Ansprüche 9 bis 11, wobei
das Flüssigkeitsmischsystem (2) ferner ein drittes Ventil (31) umfasst, das im zweiten Flussweg (25) angeordnet ist; und/oder
der zweite Flussweg (25) so konfiguriert ist, dass er das Befüllen des Kanisters (21) mit der zweiten Flüssigkeit über die Unterseite (212) des Kanisters (21) ermöglicht.

13. Das Flüssigkeitsmischsystem (2) nach einem der Ansprüche 9 bis 12, wobei das Flüssigkeitsmischsystem (2) ferner umfasst:
eine Lösungspumpe (32), die im dritten Flussweg (26) angeordnet ist, um die Lösung in die Mischkammer (22) zu pumpen; und/oder
ein viertes Ventil (33), vorzugsweise ein Rückschlagventil, das im vierten Flussweg (27) angeordnet ist.

14. Das Flüssigkeitsmischsystem (2) nach einem der Ansprüche 9 bis 13, wobei
mindestens zwei von dem ersten Flussweg (23), dem zweiten Flussweg (25) und dem vierten Flussweg (27) in Fluidkommunikation mit einer gemeinsamen Flüssigkeitsquelle (24) stehen; und/oder
mindestens eine der ersten Flüssigkeit, der zweiten Flüssigkeit und der dritten Flüssigkeit Umkehrosmosewasser ist; und/oder
die Substanz in Form von Pulver, Granulat, Tabletten oder Mischungen davon vorliegt; und/oder
die Substanz Natriumchlorid und/oder Bikarbonat, insbesondere Natriumbikarbonat, umfasst.

15. Ein Verfahren zur Herstellung einer Lösung unter Verwendung des Druckbehälters (1) nach einem der Ansprüche 1 bis 6 oder des Flüssigkeitsmischsystems (2) nach einem der Ansprüche 7 bis 14, umfassend:
Erkennen des Vorhandenseins von Luft innerhalb der Kammer (11) des Druckbehälters (1);
Ablassen der Luft durch den Druckbehälter (1) oder aus dem Flüssigkeitsmischsystem (2) im Falle des Vorhandenseins von Luft; und
Durchführen der Flüssigkeitszufuhr durch den Druckbehälter (1), um die Lösung zu erhalten.

## Revendications

1. Un récipient capable de supporter la pression (1) pour le mélange de liquides comprenant :
- un boîtier (16);
- un élément déformable (111) configuré pour définir une chambre (11) en combinaison avec au moins une partie du boîtier (16);
- un port d'entrée (12) en communication fluidique avec la chambre (11);
- un port de sortie (13) en communication fluidique avec la chambre (11);
- un moyen de précontrainte élastique (14) configuré pour interagir avec l'élément déformable (111) afin de rendre variable un volume de la chambre (11); et
- un moyen de détection d'air (15) configuré pour détecter la présence d'air à l'intérieur de la chambre (11).

2. Le récipient capable de supporter la pression (1) selon la première revendication, où
- l'élément déformable (111) est constitué d'un diaphragme flexible; et/ou
- le port d'entrée (12) et le port de sortie (13) sont disposés à l'opposé l'un de l'autre.

3. Le récipient capable de supporter la pression (1) selon la revendication 1 ou 2, où
- le moyen de précontrainte élastique (14) comprend un ressort (141), de préférence un ressort hélicoïdal conique, ou est configuré sous forme de gaz comprimé, de préférence sous forme d'air comprimé; et/ou
- le moyen de détection d'air (15) comprend une paire d'électrodes de détection d'air (151) s'étendant dans la chambre (11).

4. Le récipient capable de supporter la pression (1) selon l'une quelconque des revendications 1 à 3, où
- un espace intérieur de la chambre (11) est configuré pour générer un flux de fluide afin de faciliter le contrôle de l'hygiène dans un procédé de nettoyage et/ou de désinfection; et/ou
- le moyen de précontrainte élastique (14) est réglable de façon à réguler le volume de la chambre (11) en fonctionnement.

5. Le récipient capable de supporter la pression (1) selon la revendication 4, où
- l'espace intérieur de la chambre (11) est comprimé dans une certaine mesure par une force du moyen de précontrainte élastique (14) à un stade initial;
- de préférence, l'espace intérieur de la chambre (11) est configuré pour comporter initialement une section en forme de U le long d'une direction du moyen de précontrainte élastique (14) agissant sur l'élément déformable (111) et prolongeant le port d'entrée (12) ainsi que le port de sortie (13), et une partie inférieure (112) de la section en forme de U est définie par l'élément déformable (111) .

6. Le récipient capable de supporter la pression (1) selon la revendication 5, où
- le moyen de détection d'air (15) est adjacent à une extrémité extérieure (1131) d'une première branche (113) de la section en forme de U; et/ou
- le port d'entrée (12) est adjacent à un angle (114) entre la première branche (113) et la partie inférieure (112) de la section en forme de U; et/ou
- le port de sortie (13) est adjacent à un point médian d'une seconde branche (115) de la section en forme de U opposée à la première branche (113).

7. Un système de mélange de liquides (2) comprenant le récipient capable de supporter la pression (1) selon l'une quelconque des revendications 1 à 6.

8. Le système de mélange de liquides (2) selon la revendication 7, où le système de mélange de liquides (2) comprend en outre :
- un réservoir (21), notamment un réservoir rigide, pour recevoir une substance à dissoudre ou à diluer par un premier liquide afin de préparer une solution, de préférence une solution saturée, où le port de sortie (13) du récipient capable de supporter la pression (1) est relié de manière fluidique au réservoir (21); et
- une chambre de mélange (22) reliée de manière fluidique au récipient (21) pour recevoir la solution;
- de préférence, le port d'entrée (12) du récipient capable de supporter la pression (1) est positionné plus haut que le port de sortie (13) du récipient capable de supporter la pression (1) en état de fonctionnement.

9. Le système de mélange de liquides (2) selon la revendication 8, où le système de mélange de liquides (2) comprend en outre :
- un premier chemin d'écoulement (23) relié de manière fluidique au port d'entrée (12) du récipient capable de supporter la pression (1) pour permettre le remplissage du réservoir (21) avec le premier liquide via le premier chemin d'écoulement (23) et le récipient capable de supporter la pression (1); et/ou
- un second chemin d'écoulement (25) relié de manière fluidique au réservoir (21) pour permettre le remplissage du réservoir (21) avec un second liquide; et/ou
- un troisième chemin d'écoulement (26) relié de manière fluidique au réservoir (21) pour permettre le transfert de la solution vers la chambre de mélange (22); et/ou
- un quatrième chemin d'écoulement (27) relié de manière fluidique à la chambre de mélange (22) pour fournir un troisième liquide à la chambre de mélange (22).

10. Le système de mélange de liquides (2) selon la revendication 9, où le système de mélange de liquides (2) comprend en outre :
- un moyen de détection de pression (28) configuré pour détecter une pression à l'intérieur du premier chemin d'écoulement (23);
- une première vanne (29) disposée dans le premier chemin d'écoulement (23), en amont du moyen de détection de pression (28); et
- un dispositif d'évacuation d'air (30) relié de manière fluidique au premier chemin d'écoulement (23) entre la première vanne (29) et le récipient capable de supporter la pression (1).

11. Le système de mélange de liquides (2) selon la revendication 10, où le dispositif d'évacuation d'air (30) comprend une seconde vanne (301) ainsi qu'une sortie d'air (302); et/ou
- le réservoir (21) possède un dessus (211) et un dessous (212) en état de fonctionnement, et le récipient capable de supporter la pression (1) est relié de manière fluidique à un port d'entrée (213) situé en haut du réservoir (21) pour permettre le remplissage du réservoir (21) avec le premier liquide par le dessus (211) du réservoir (21).

12. Le système de mélange de liquides (2) selon l'une quelconque des revendications 9 à 11, où
- le système de mélange de liquides (2) comprend en outre une troisième vanne (31) disposée dans le second chemin d'écoulement (25); et/ou
- le second chemin d'écoulement (25) est configuré pour permettre le remplissage du réservoir (21) avec le second liquide par le dessous (212) du réservoir (21).

13. Le système de mélange de liquides (2) selon l'une quelconque des revendications 9 à 12, où le système de mélange de liquides (2) comprend en outre :
- une pompe à solution (32) disposée dans le troisième chemin d'écoulement (26) pour pomper la solution vers la chambre de mélange (22); et/ou
- une quatrième vanne (33), de préférence une vanne anti-retour, disposée dans le quatrième chemin d'écoulement (27).

14. Le système de mélange de liquides (2) selon l'une quelconque des revendications 9 à 13, où
- au moins deux chemins d'écoulement parmi le premier chemin d'écoulement (23), le second chemin d'écoulement (25) et le quatrième chemin d'écoulement (27), sont reliés de manière fluidique à une source de liquide commune (24); et/ou
- au moins un des trois liquides, le premier liquide, le second liquide et le troisième liquide, est de l'eau obtenue par osmose inverse; et/ou
- la substance se présente sous forme de poudre, de granulés, de comprimés ou de mélanges de ceux-ci; et/ou
- la substance comprend du chlorure de sodium et/ou du bicarbonate, notamment du bicarbonate de sodium.

15. Un procédé de préparation d'une solution à l'aide du récipient capable de supporter la pression (1) selon l'une quelconque des revendications 1 à 6 ou le système de mélange de liquides (2) selon l'une quelconque des revendications 7 à 14, comprenant les étapes consistant à :
- détecter la présence d'air dans la chambre (11) du récipient capable de supporter la pression (1);
- évacuer l'air via le récipient capable de supporter la pression (1) ou hors du système de mélange de liquides (2) en cas de présence de l'air; et
- alimenter en liquide via le récipient capable de supporter la pression (1) pour obtenir la solution.
